# EUROPÄISCHE PATENTANMELDUNG

(11) **EP 0 950 422 A1**
(43) Veröffentlichungstag der Anmeldung: **20.10.1999**
(21) Anmeldenummer: 98810334.7
(22) Anmeldetag: 17.04.1998
(51) Int. Cl.: A61M 1/28, A61B 19/02

(54) **Dialysatwechsel-Ausrüstung für Peritonealdialyse-Patienten**

(71) Anmelder: Matzke, Laura, 9470 Buchs (CH)
(72) Erfinder: Matzke, Laura, 9470 Buchs (CH)
(74) Vertreter: Riederer, Conrad A., Dr.

(57) **Zusammenfassung**

Bei einer Ausrüstung für Peritonealdialyse-Patienten (11), um den Dialysatwechsel vorzunehmen, sind die Verbrauchsmaterialien wie Händedesinfektionsmittel (53), Alkohol (55) zur Reinigung der Arbeitsfläche, Gesichtsmasken (33), Hygienetücher (29), und sterile Verschlusskappen wie Schutzmuffen etc. in einem Koffer (23a) angeordnet. Im selben Koffer (23a) ist eine Arbeitsplatte (21a) verstaubar und sind die Dialysatbeutel (19) für einen Tagesbedarf vorrätig. Die Dialysatbeutel (19 sind in einem heizbaren und wärmegedämmten Abteil (25a). Die Arbeitsplatte (21a) weist Kissen (63) auf ihrer Unterseite auf, mit welchen sie auf en Oberschenkeln des Patienten (11) Halt findet. Die Arbeitsplatte (21a) dient als Arbeitsfläche beim Dialysatwechsel unterwegs.

## Beschreibung

### Technisches Gebiet der Erfindung

Die vorliegende Erfindung betrifft eine Dialysatwechsel-Ausrüstung für Peritonealdialyse-Patienten mit einem Dialysatbeutel, einem Auslaufbeutel und dazugehörigen Überleitungsschläuchen und einem Konnektor zum Anschliessen an den Bauchkatheter des Patienten, und mit beim Dialysatwechsel benötigten Verbrauchsmaterialien und Hilfsmitteln. Die Erfindung betrifft insbesondere eine Dialysatwechsel-Ausrüstung für den Dialysatwechsel auf Reisen.

### Stand der Technik

Dialysepatienten haben die Wahl zwischen Hämodialyse und Peritonealdialyse. Für die Hämodialyse muss der Patient regelmässig an eine künstliche Niere angeschlossen werden. Dazu muss er in der Regel ein Dialysezentrum aufsuchen, wo der Blutkreislauf an die künstliche Niere angeschlossen wird und ihm so das Blut gereinigt wird.

Bei der Peritonealdialyse wird eine spezielle Flüssigkeit, das Dialysat, über einen Katheter in die Bauch- und Beckenhöhle oder Peritonealhöhle eingefüllt, wo es in ständigem Kontakt mit dem Bauchfell oder Peritoneum ist. Die Peritonealdialyse nutzt dabei die Erkenntnis, dass das Bauchfell eine grosse, gut durch blutete Haut ist, über welche durch Osmose Wasser dem Blutkreislauf entzogen werden kann und durch Diffusion Giftstoffe aus dem Blut in das Dialysat gelangen können. Das mehr und mehr mit Giftstoffen angereicherte Dialysat wird nach einer gewissen Zeit durch neues ersetzt.

Die Peritonealdialyse bietet den Vorteil, dass der Patient in irgendeinem Zimmer, sei es bei sich zuhause, im Büro oder in einem Hotelzimmer, selbständig einen Dialysatwechsel vornehmen kann. Dieser Dialysatwechsel muss tagsüber 3-mal bis 5-mal durchgeführt werden. Die Peritonealdialyse eröffnet dem Patienten die Möglichkeit, sich auch auf Reisen zu begeben, muss er doch lediglich innerhalb von rund vier Stunden wieder ein Zimmer für sich haben. Das Vorplanen von Aufenthalten in Dialysezentren ist nicht notwendig. Zudem kann auch ohne grosse Schwierigkeiten ein Dialysatwechsel hinausgedehnt oder gar ausgelassen werden. Dadurch ist die Beweglichkeit des Patienten deutlich erhöht.

Für den Dialysatwechsel wird vorgeschrieben, dass er bei geschlossenen Fenstern, in einem zugluftfreiem Raum, unter sehr hygienischen Bedingungen stattfindet. Der Patient und allfällige Helfer tragen dazu eine Gesichtsmaske, die Hände werden mehrfach desinfiziert. Der Wechsel wird an einem Tisch durchgeführt. Die Tischfläche wird vorbereitend mit Alkohol desinfiziert. Eine Schutzmuffe für die Verbindungsstelle zwischen Konnektor und Katheter, eine sterile Verschlusskappe für den Katheter und ein frischer Dialysatbeutel werden bereitgelegt. Nach der Händedesinfektion wird der auf Körpertemperatur vorgewärmte Dialysatbeutel kontrolliert und auf die Tischfläche gelegt. Die Ein- und Auslaufschläuche werden so unter dem Beutel eingeklemmt, dass der Konnektor ca. 10 bis 15 cm über den Tischrand vorsteht. Nachdem der Katheter aus den Kleidern hervorgenommen und bereitgelegt ist, werden die Hände nochmals desinfiziert und die Schutzhülle der Schutzmuffe vorsichtig geöffnet. Nun wird der Konnektor freigelegt und die Verschlusskappe des Katheters entfernt und sofort das Beutelsystem mit dem Katheter verbunden. Mit der Schutzmuffe wird die Verbindungsstelle geschützt. Mit einer Klemme wird der Schlauch des vollen Beutels abgeklemmt. Nun wird die Rollklemme am Katheter geöffnet. Nach Auslaufen der Flüssigkeit wird der Schlauch des Auslaufbeutel abgeklemmt, die Rollklemme am Katheter geschlossen, der den Schlauch verschliessende Dorn im Schlauch des Dialysatbeutels gebrochen, die Klemme am selben Schlauch und die Rollklemme am Bauchkatheter geöffnet. Für das Auslaufenlassen und das Einlaufenlassen des Dialysats werden etwa 20 bis 30 Minuten benötigt. Während des Wechsels, d.h. während des Auslaufens des verbrauchten Dialysats in den Auslaufbeutel und während des Einlaufens des neuen Dialysats ist der Patient relativ frei. So kann er sich frei in einer Gesellschaft bewegen, Arbeiten, sich ausruhen oder tun, was er zu tun wünscht. Er muss lediglich den Beutel mit sich führen; beim Auslaufenlassen muss der Auslaufbeutel in tieferer Lage als der Bauchraum, beim Einlaufenlassen der Dialysatbeutel in höherer Lage als der Bauchraum mitgeführt werden. Danach können die Beutel wieder vom Katheter abgehängt werden, wozu wieder diese hygienischen Verhältnisse vorliegen müssen.

Zum Abhängen der Beutel nach Einlaufen des neuen Dialysats werden der Schlauch und der Katheter zugesperrt und bei aufgesetztem Mundschutz die Hände desinfiziert. Dann wird eine neue Verschlusskappe für den Katheter auf dem Tisch bereitgelegt, die Verbindung zwischen Schlauch und Katheter gelöst und der Katheter mit der sterilen Verschlusskappe verschlossen und in den Kleidern versorgt. Das verbrauchte Dialysat und die alten Beutel können nun entsorgt werden.

Beim Wechsel sind die kritischen Momente das Entfernen der Verschlusskappe des Bauchkatheters und das anschliessende Verbinden der Schläuche mit dem Katheter wie auch das Abhängen der Schläuche und das erneute Verschliessen des Bauchkatheters. Bei diesen Handlungen darf es zu keinerlei Kontakt zwischen den Verbindungsteilen und einer nicht sterilen Oberfläche kommen. Zur Erhöhung der Sicherheit beim Zusammenführen und Lösen von Konnektor und Katheter und beim Verschliessen des Katheters werden vorzugsweise die Hände auf einem Tisch aufgestützt. Deshalb werden diese Handlungen sitzend an einem vorbereiteten Tisch, gewöhnlich am eigens dafür vorgesehenen Platz in der eigenen Wohnung vorgenommen.

Es ist deshalb Aufgabe der Erfindung eine Ausrüstung für Dialysepatienten zu schaffen, welche den Dialysatwechsel auch im Auto, im Zug oder Flugzeug erlaubt. Diese Möglichkeit, insbesondere der Wechsel im Auto, erhöht die Bewegungsfreiheit des Dialysepatienten wesentlich. Er kann dadurch einen ganzen Tag unterwegs sein, und erst am Abend z.B. im Hotel ankommen, wo er wieder an einem Tisch sitzend den Dialysatwechsel vornehmen kann.

### Beschreibung der Erfindung

Erfindungsgemäss weist eine Dialysatwechsel-Ausrüstung für Peritonealdialyse-Patienten einen Koffer zum Aufnehmen der für den Dialysatwechsel notwendigen Verbrauchsmaterialien auf. Erfindungswesentlicher Bestandteil der Ausrüstung ist zudem eine Arbeitsplatte, welche im/oder am Koffer ihren Platz hat, auf den Oberschenkeln einer sitzenden Person Halt findet und eine Arbeitsfläche zum Manipulieren bietet, und welche Platz für die bei einem Dialysatwechsel benötigten Gegenstände aufweist, insbesondere für eine Schutzmuffe für den Konnektor bzw. eine sterile Verschlusskappe für den Bauchkatheter.

Im Koffer sind die notwendigen Verbrauchsmaterialien und allenfalls Hilfsmittel angeordnet, wie z.B. eine Waschlotion für die Reinigung der Hände, Sterilium für die Händedesinfektion, Alkohol für die Reinigung der Arbeitsplatte, Gesichtsmasken, steril verpackte Verschlusskappen und Schutzmuffen, Hygienetücher für die Reinigung von Händen und Arbeitsfläche, zwei Schlauchklemmen und ein Haken zum Aufhängen des Dialysatbeutels. Dadurch ist alles notwendige Material beim Wechsel zur Hand. Es kann nun die Arbeitsplatte auf die Oberschenkel gelegt werden, allenfalls daran befestigt werden, die Verbrauchsmaterialien und Hilfsmittel sowie ein neuer Dialysatbeutel darauf vorbereitet und wie am Tisch zuhause der Wechsel vorgenommen werden. Im Auto wird dazu sicherheitshalber angehalten, Motor und Lüftung abgestellt, und der Beutel angeschlossen. Während des Ein- und Auslaufens kann die Reise fortgesetzt werden. Da die Höhendifferenz zwischen Bauchhöhle und Auslaufbeutel im Auto in der Regel niedriger ist als zuhause, und da auch der Dialysatbeutel nicht sehr hoch über der Bauchhöhle aufgehängt werden kann, dauert das Auslaufen und Einlaufen etwas länger als sonst, nämlich ca. 40 Minuten.

Die Dialysatbeutel können in einem separaten Behälter, z.B. einem zweiten Koffer mitgeführt werden. Für längere Aufenthalte müssen ohnehin die Beutel in grösseren Mengen mitgeführt werden. Vorteilhaft enthält der Koffer jedoch zwei Abteile, wobei in einem Abteil Platz für die Hilfsmittel und Verbrauchsmaterialien und im andern für mehrere Dialysatbeutel mit den dazugehörigen Auslaufbeuteln, Überleitungsschläuchen und Konnektoren vorhanden ist. Vorzugsweise ist darin Platz für drei Dialysatbeutel. Dadurch ist im Koffer Platz für eine Tagesration. Diese kann einmal täglich, gewöhnlich abends, nachgefüllt werden.

Damit die Beutel über 12 Stunden auf etwa Körpertemperatur bleiben, ist das Abteil für die Dialysatbeutel vorteilhaft wärmegedämmt.

Wenn das Abteil für die Dialysatbeutel heizbar ist, können die kalten Beutel in das Abteil nachgefüllt werden und z.B. über Nacht darin aufgeheizt werden.

Vorteilhaft ist die Arbeitsplatte auf der Unterseite mit einem Kissen ausgerüstet, welches auf die Form von Oberschenkeln angepasst oder anpassbar ist. Das Kissen kann dazu zwei gewölbeförmige Einbuchtungen aufweisen. Alternativ dazu kann es auch ein Sack mit einer sich an praktisch jegliche Untergrundform anpassenden Füllung sein. Solche Füllkörner sind z.B. aus der Möbelindustrie bekannt. Dadurch findet die Arbeitsplatte auf den Oberschenkeln einer Person halt.

Vorteilhaft ist an der Arbeitsplatte eine Halterung für die Überleitungsschläuche bzw. den Konnektor vorgesehen. Da um die Arbeitsplatte herum im Auto oder in einem anderen Verkehrsmittel gewöhnlich wenig Platz besteht, kann der Konnektor nicht ohne ein gewisses Kontaminationsrisiko über den Plattenrand vorstehen gelassen werden. Selbst an einem Tisch ist der überstehende und ungeschützte Konnektor gefährdet. Durch die Verwendung einer Halterung kann der Konnektor über der Arbeitsplatte an einer definierten und allenfalls sogar geschützten Stelle festgehalten werden. Vorteilhaft ist an der Arbeitsplatte auch eine Halterung für den Katheter vorgesehen. Diese zwei Halterungen sind vorzugsweise so angeordnet und verschiebbar, dass die Verbindungsstücke einander gegenüber liegen und mit der Halterung zusammengeführt werden können. Dadurch ist die risikoreichste Handlung beim Dialysatwechsel geführt und daher abgesichert.

Vorteilhaft sind auf der Arbeitsplatte Halterungen und/oder Mulden für die benötigten Verbrauchsmaterialien und Hilfsmittel vorgesehen. Dadurch werden diese jeweils an der richtigen Stelle bereitgelegt und es wird nichts vergessen, was, wenn nicht ordnungsgemäss vorbereitet, den eingespielten und zweckmässigen Ablauf unterbrechen könnte.

Bei einer bestimmten Ausführungsform einer Dialysatwechsel-Ausrüstung bilden die Arbeitsplatte und das Abteil für die Hilfsmittel und Verbrauchsmaterialien eine beim Dialysatwechsel auf die Oberschenkel gelegte und von einem Behälter für die Dialysatbeutel getrennte Einheit. Bei dieser Einheit haben vorzugsweise einige Verbrauchsmaterialien und Hilfsmittel ihren festen Platz im Abteil und andere ihre festen Platz auf der Arbeitsplatte. So haben beispielsweise im etwa senkrecht aufgestellten oder beispielsweise gegen den Vordersitz angelehnten Abteil für die Hilfsmittel und Verbrauchsmaterialien die Fläschchen mit Reinigungslotion, Sterilium und Alkohol, der Haken, die Schutzmasken und Hygienetüchlein und der Vorrat an Sterilen Verschlusskappen und Schutzmuffen ihren Platz, auf der Arbeitsfläche aber ist eine Mulde für die Verpackung von Schutzmuffe bzw. Verschlusskappe, die Halterung für den Konnektor und allenfalls den Katheter und z.B. eine Mulde für den Dialysatbeutel vorgesehen. Dadurch bleibt mehr Platz für die Manipulation vorhanden und die verschiedenen Fläschchen und Utensilien sind sofort nach dem Öffnen des Abteils zur Hand.

Zweckmässigerweise ist das Abteil für die Hilfsmittel und Verbrauchsmaterialien im Kofferdeckel, das Abteil für die Dialysatbeutel im Kofferboden und die Arbeitsplatte zwischen den beiden Abteilen angeordnet.

Vorteilhaft ist über der Arbeitsplatte, allenfalls zwischen dem Abteil mit den Hilfsmitteln und Verbrauchsmaterialien und der Arbeitsplatte, eine transparente Schutzhülle angeordnet. Dadurch ist der Arbeitsbereich während der Manipulationen für den Dialysatwechsel vor Zugluft und Umwelteinflüssen weitgehend geschützt und damit das Infektionsrisiko weiter gesenkt. Vorteilhaft ist eine Lichtquelle für ultraviolettes Licht im Koffer vorgesehen, welche die Arbeitsplatte und die Verbrauchsmaterialien und Hilfsmittel mit keimtötendem Licht beleuchten kann. Diese kann zur Desinfektion von Arbeitsplatte, Halterungen Utensilien etc. z.B. über nacht eingeschaltet werden, aber auch während der Manipulation eingeschaltet sein und dadurch die Kontamination mit aktiven Keimen aus der Luft verringern.

### Kurzbeschrieb der Figuren

Es zeigt:
- Fig. 1: zeigt einen Dialysepatienten mit einem erfindungsgemässen Koffer beim Anschliessen eines Dialysatbeutels an seinen Peritonealkatheter,
- Fig. 2: eine Arbeitsplatte mit Haltevorrichtungen für den Katheter und die Überleitungsschläuche,
- Fig. 3: einen Dialysatwechsel-Koffer mit abnehmbarem Deckel und einer Arbeitsplatte mit einer Beutelmulde,
- Fig. 4: die Arbeitsplatte mit Schutzhülle und Kofferdeckel des Dialysatwechsel-Koffers gemäss Figur 5 und 6,
- Fig. 5: einen Querschnitt durch den geschlossenen Koffer,
- Fig. 6: einen Längsschnitt durch den geschlossenen Koffer gemäss Figur 5,
- Fig. 7: einen Schnitt durch eine Arbeitsplatte gemäss Figur 2 mit zwei Halterungen für den Katheter und die Überleitungsschläuche in einer Führungsschiene.

### Ausführlicher Beschrieb der Ausführungsbeispiele anhand der Figuren

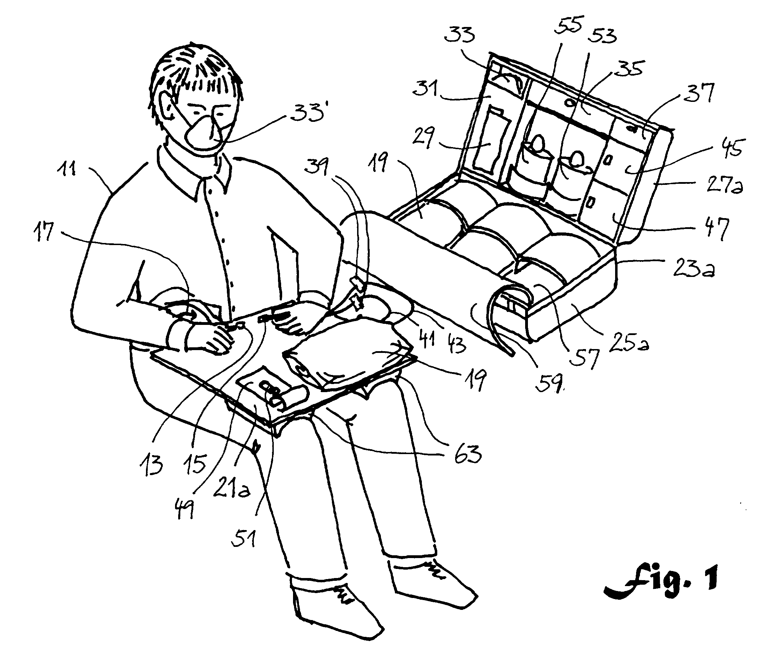

Der Dialysepatient 11 in Figur 1 ist dabei, die Verbindungsteile 13 und 15 seines Peritonealkatheters 17 und eines Dialysatbeutels 19 zusammenzuführen. Dazu hat er seine Hände auf der Arbeitsplatte 21a abgestützt. Die Arbeitsplatte 21a liegt auf seinen Oberschenkeln. Neben sich hat er den Koffer 23a mit den Dialysatbeuteln 19 im Bodenteil 25a. Im Deckelteil 27a des Koffers 23a sind die Verbrauchsmaterialien und Hilfsmittel untergebracht und jederzeit verfügbar.

Links im Deckel 27a liegen Hygienetüchlein 29 in einem Spender 31 vor, darüber in einer Nische sind einige Gesichtsmasken 33 vorhanden, von denen der Mann 11 sich eine (33') aufgesetzt hat. Im oberen mittleren Fach 35 mit einem mit Federkraft selbstschliessenden Klapptürchen sind eine Händereinigungslotion oder eine Seife und eine Handbürste untergebracht. Im Fach 37 daneben können die beiden Schlauchklemmen 39 eingeräumt werden, welche die Überleitungsschläuche 41 und 43 abklemmen.

In den Fächern 45 und 47 am rechten Rand des Kofferdeckels 27a sind die Päckchen mit den sterilen Schutzmuffen und Verschlussdeckeln untergebracht. Ein solches Päckchen 49 mit einer Schutzmuffe 51 liegt geöffnet auf der Arbeitsplatte 21a. In der Mitte des Kofferdeckels 27a sind das Sterilium-Fläschchen 53 und das Alkoholfläschchen 55 in Taschen eingesteckt. Die Dialysatbeutel 19 liegen in Fächern 57 im Kofferboden 25a und sind durch eine wärmedämmende Decke 59 zudeckbar.

Zwischen dem Bodenteil 25a und dem Deckelteil 27a kann die Arbeitsplatte 21a eingeräumt werden. Auf der Unterseite der Arbeitsplatte sind zwei Kissen 63 angebracht, welche eine konkave Rundung aufweisen, um auf den Oberschenkeln des Patienten 11 Halt zu finden.

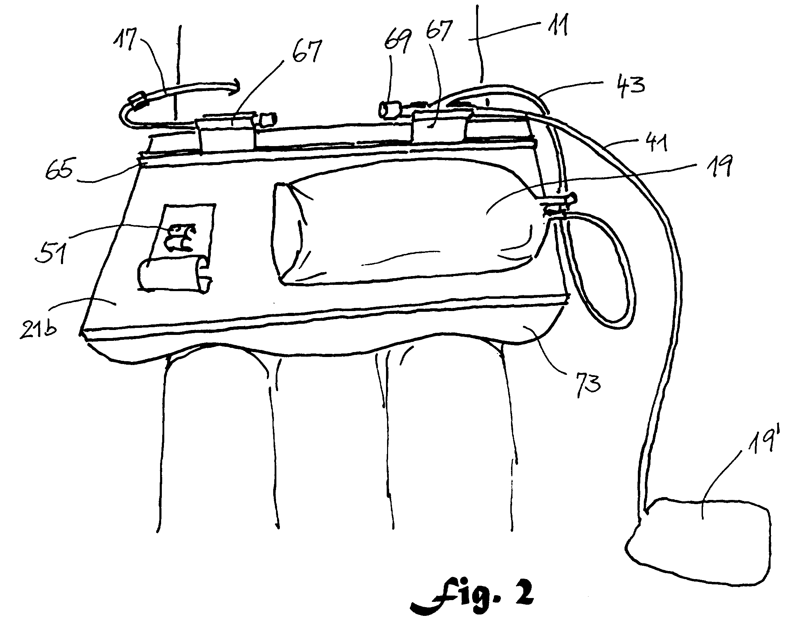

Figur 2 zeigt eine Arbeitsplatte 21b, welche eine Führungssschiene 65 und darin zwei Halter 67 für die zu verbindenden Schläuche, den Katheter 17 und die Überleitungsschläuche 41,43 bzw. den Konnektor 69. Sind die beiden Kupplungsstücke, Katheterkopf 71 und Konnektor 69 von ihren Schutzkappen befreit, können sie exakt zusammengeführt werden, indem die Halter 67 in der Führungsschiene 65 gegeneinander verschoben werden. Die Schutzmuffe 51 liegt bereit, um auf die zusammengeschraubten Kupplungsstücke aufgesetzt zu werden. Die Arbeitsplatte 21b weist auf ihrer Unterseite ein formbares Kissen 73 auf. Das Kissen ist aus einem Sack mit einem Granulat darin hergestellt, welches Granulat sich an die Form der Oberschenkel anpasst.

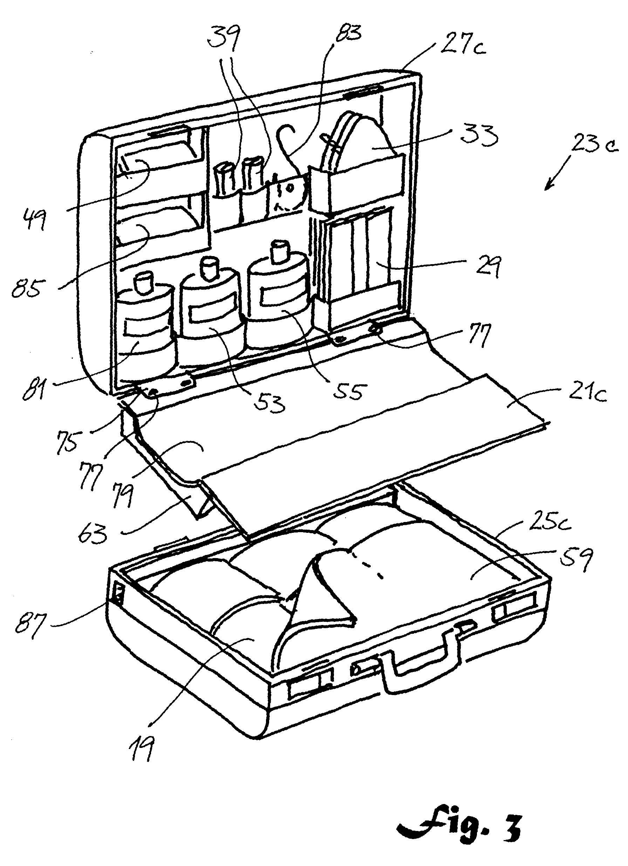

Figur 3 zeigt einen Dialysatwechsel-Koffer 23c, bei welchem der Deckel 27c vom Bodenteil 25c ablösbar ist. Die Arbeitsplatte 21c ist mit dem Deckelteil 27c über Laschen 75 verbunden. Die Laschen 75 sind mittels Druckknöpfen 77 an der Arbeitsplatte 21c lösbar befestigt. Die Arbeitsplatte 21c weist Kissen 63 an ihrer Unterseite auf. Sie weist eine Mulde 79 zur Aufnahme eines Dialysatbeutels 19 auf. Im Kofferdeckel 27c sind eine Waschlotion 81, ein Fläschchen 53 mit Händedesinfektionsmittel und ein Fläschchen 55 mit Alkohol zur Reinigung der Arbeitsfläche und dergleichen untergebracht. Weiter sind in entsprechenden Fächern und Taschen Hygienetüchlein 29, Gesichtsmasken 33, ein Haken 83 zum Aufhängen des neuen Dialysatbeutels 19 während des Einlaufenlassens, zwei Klemmen 39 und die Päckchen 49 und 85 für Schutzmuffen und Verschlusskappen eingeräumt.

Im Kofferboden 25c ist ein ausziehbarer Stecker 87 vorgesehen. An diesem Stecker ist eine elektrische Heizung der Dialysatbeutel 19 angeschlossen.

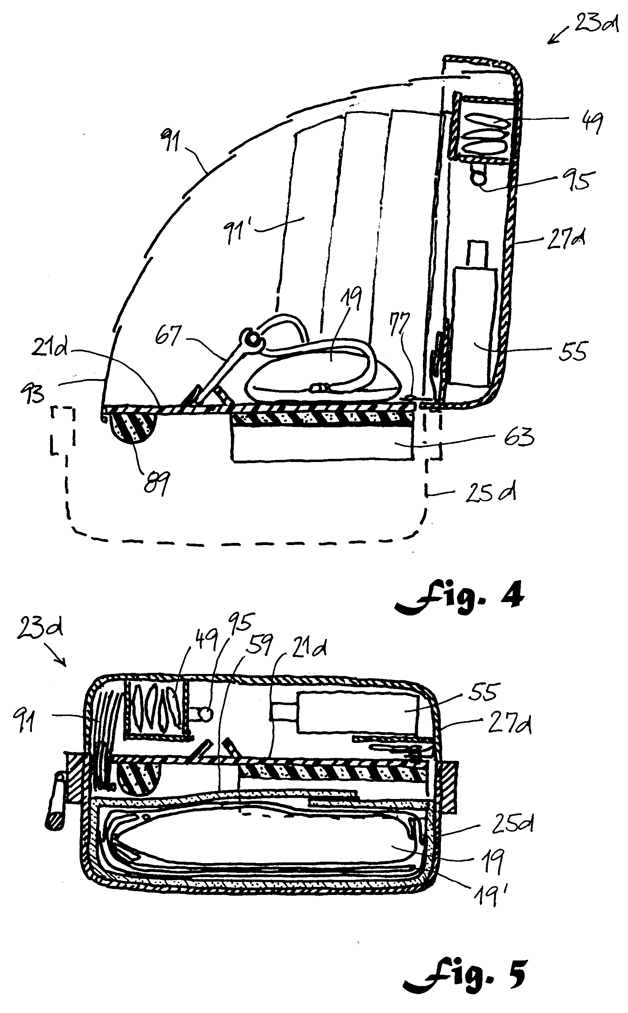

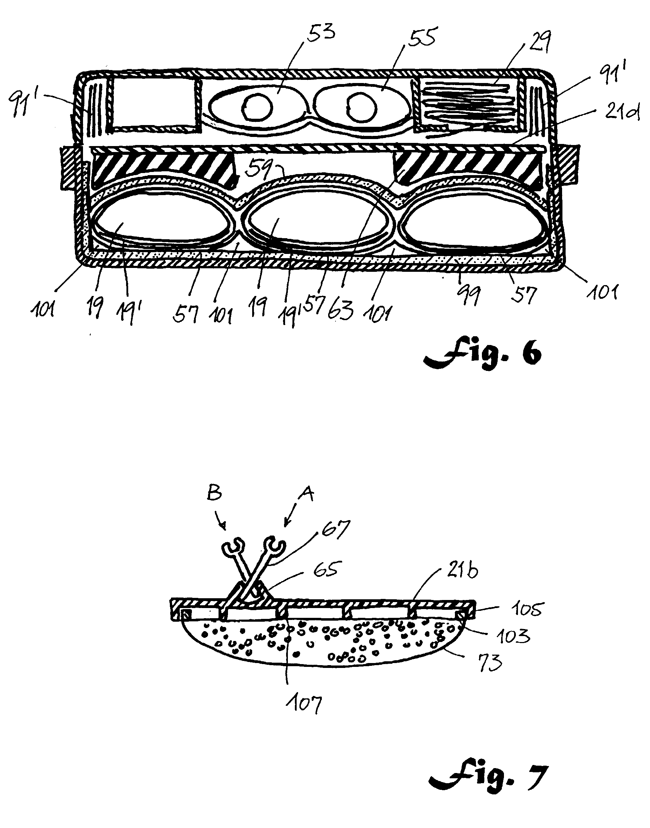

Figuren 4 bis 6 zeigen einen Koffer 23d, bzw. Figur 4 die Arbeitsplatte 21d und den Kofferdeckel 27d im Schnitt. Der Kofferboden 25d ist mit unterbrochenen Linien dargestellt. Figur 5 zeigt einen Querschnitt und Figur 6 eine Längsschnitt durch den Koffer 23d. Die Arbeitsplatte 21d ist wiederum lösbar. Sie ist mit einem Kissen 63 mit einer konkaven Form und einem entlang der bauchseitigen Kante der Arbeitsplatte 21c langgestrecken Kissen 89 ausgerüstet.

Der Koffer 23c ist zudem mit einer Schutzhülle 91 ausgerüstet. Diese besteht aus einer Vielzahl von Lamellen, welche übereinander verschoben werden können. Sei sind seitlich ineinandergesteckt, so dass jeweils die obere Lamelle die untere seitlich umfasst. Dadurch ist die unterste Lamelle 93 die schmalste. Diese ist unter der Arbeitsplatte 21c eingehängt. Dadurch wird die Schutzhülle 91 aufgeklappt, wenn de Deckel 27c geöffnet wird. Seitlich ist in ähnlicher Konstruktion eine Blende 91' aus dem Kofferdeckel 27c hervorziehbar. Dank dem die Schutzhülle unten schmaler ist als oben, kann mit den Händen gut hinter die Schutzhülle 91 gegriffen werden und im Schutze der transparenten Lamellen gearbeitet werden.

Weiter ist der Koffer 23c mit einer ultravioletten Lichtquelle 95 versehen. Diese kann bei geöffnetem Koffer 23d eingeschaltet werden, um keimtötend auf den Arbeitsbereich, d.h. die Arbeitsplatte 21d und den inneren Bereich des Kofferdeckels 27d zu wirken. Diese Wirkung kann auch während dem Ankoppeln und Abkoppeln des Dialysatbeutels erwünscht sein.

Aus der Figur 6 ist die Heizeinrichtung 97 für die Dialysatbeutel 19 schematisch ersichtlich. Innerhalb der Wärmedämmung 99,59 sind drei Mulden oder Fächer 57 für die Dialysatbeutel 19 vorgesehen. Zwischen diesen ist jeweils eine Abgrenzung 101 oder ein Muldenrand ausgebildet. Im Innern dieses Randes 101 sind Heizelemente angeordnet. Es kann auch eine die ganze Fläche, auf denen die Beutel 19 aufliegen, abdeckende Heizung vorgesehen sein.

In Figur 7 ist ein Querschnitt durch eine Arbeitsplatte gemäss Figur 2 dargestellt. Die Arbeitsplatte 21b ist mit einem verformbaren Kissen 73 auf seiner Unterseite versehen. Die Füllung füllt den maximalen Innenraum des Kissens nicht völlig aus, damit es sich unter Verdrängung der Luft im Kissen auf die Oberschenkel des Benutzers anpassen kann. Das Kissen ist aus einer locker unter die Arbeitsplatte gehängte Stoffbahn gefertigt. Dazu ist ein innerer Rahmen 103 in einen mit der Arbeitsplatte verbundenen äusseren Rahmen 105 geklemmt. Die Arbeitsplatte 21b ist auf ihrer Unterseite mit Versteifungsrippen 107 und auf der Oberseite mit einer Führungsschiene 65 versehen.

Die Führungsschiene 65 besteht aus zwei parallelen niedrigen Wänden, welche gegeneinander geneigt sind. Scheiben oder Stäbe der Halterungen 67 für die Schläuche sind in die Führungsschiene 65 einsteckbar. Dank der Neigung der Wände der Führungsschiene 65 lassen sich die Halterungen 67 auf zwei Seiten kippen. Die Halterungen, bzw. ihre Scheiben oder Stäbe sind jeweils mit der Innenfläche der einen Wandung und der Stirnfläche der andern Wandung in Kontakt. Dadurch kann die Halterung um etwa 60 bis 90 Grad gekippt werden. Ist die eine Halterung von der die Arbeitsplatte 21b benutzenden Person weggekippt (Position A) und die andere zu ihr hin (Position B), so kann an der zu ihr hingekippten Halterung 67 manipuliert werden, z.B. zum Entfernen der Verschlusskappe des Katheters, ohne dass der in der anderen Halterung eingesteckte Schlauch in dem Manipulatonsbereich hineinsteht. Danach können beide Halterungen gekippt werden und am Ende des anderen Schlauchs gearbeitet werden. Sind beide Schlauchenden offen, werden die Halterungen auf die gleiche Seite gekippt und gegeneinander geführt und schliesslich die Verbindungsstücke zusammengeschraubt.

## Patentansprüche

1. Dialysatwechsel-Ausrüstung für Peritonealdialyse-Patienten, insbesondere für den Dialysatwechsel auf Reisen, mit einem Dialysatbeutel, einem Auslaufbeutel und dazugehörigen Überleitungsschläuchen und einem Konnektor zum Anschliessen an den Bauchkatheter des Patienten, und mit beim Dialysatwechsel benötigten Verbrauchsmaterialien und Hilfsmitteln, gekennzeichnet durch einen Koffer zum Aufnehmen der für den Dialysatwechsel notwendigen Verbrauchsmaterialien, und durch eine Arbeitsplatte, welche im/oder am Koffer ihren Platz hat, auf den Oberschenkeln einer sitzenden Person Halt findet und eine Arbeitsfläche zum Manipulieren bietet, und welche Platz für die bei einem Dialysatwechsel benötigten Gegenstände aufweist, insbesondere für eine Schutzmuffe für den Konnektor bzw. eine sterile Verschlusskappe für den Bauchkatheter.

2. Dialysatwechsel-Ausrüstung nach Anspruch 1, dadurch gekennzeichnet, dass der Koffer zwei Abteile enthält, wobei in einem Abteil Platz für die Hilfsmittel und Verbrauchsmaterialien und im andern für mehrere Dialysatbeutel mit den dazugehörigen Auslaufbeuteln, Überleitungsschläuchen und Konnektoren vorhanden ist, vorzugsweise für drei Dialysatbeutel.

3. Dialysatwechsel-Ausrüstung nach Anspruch 1 oder 2, dadurch gekennzeichnet, dass das Abteil für die Dialysatbeutel wärmegedämmt ist.

4. Dialysatwechsel-Ausrüstung nach einem der Ansprüche 1 bis 3, dadurch gekennzeichnet, dass das Abteil für die Dialysatbeutel heizbar ist.

5. Dialysatwechsel-Ausrüstung nach einem der Ansprüche 1 bis 4, dadurch gekennzeichnet, dass die Arbeitsplatte auf der Unterseite mit einem Kissen ausgerüstet ist, welches auf die Form von Oberschenkeln angepasst oder anpassbar ist.

6. Dialysatwechsel-Ausrüstung nach einem der Ansprüche 1 bis 5, dadurch gekennzeichnet, dass an der Arbeitsplatte eine Halterung für die Überleitungsschläuche bzw. den Konnektor, und allenfalls eine Halterung für den Katheter vorgesehen ist.

7. Dialysatwechsel-Ausrüstung nach einem der Ansprüche 1 bis 6, dadurch gekennzeichnet, dass auf der Arbeitsplatte Halterungen und/oder Mulden für die benötigten Verbrauchsmaterialien und Hilfsmittel vorgesehen sind.

8. Dialysatwechsel-Ausrüstung nach einem der Ansprüche 1 bis 7, dadurch gekennzeichnet, dass die Arbeitsplatte und das Abteil für die Hilfsmittel und Verbrauchsmaterialien eine beim Dialysatwechsel auf die Oberschenkel gelegte und von einem Behälter für die Dialysatbeutel getrennte Einheit bilden, wobei vorzugsweise einige Verbrauchsmaterialien und Hilfsmittel ihren festen Platz im Abteil und andere ihre festen Platz auf der Arbeitsplatte haben.

9. Dialysatwechsel-Ausrüstung nach einem der Ansprüche 2 bis 8, dadurch gekennzeichnet, dass das Abteil für die Hilfsmittel und Verbrauchsmaterialien im Kofferdeckel, das Abteil für die Dialysatbeutel im Kofferboden und die Arbeitsplatte zwischen den beiden Abteilen angeordnet ist.

10. Dialysatwechsel-Ausrüstung nach einem der Ansprüche 1 bis 9, dadurch gekennzeichnet, dass über der Arbeitsplatte, allenfalls zwischen dem Abteil mit den Hilfsmitteln und Verbrauchsmaterialien und der Arbeitsplatte, eine transparente Schutzhülle angeordnet ist.

11. Dialysatwechsel-Ausrüstung nach einem der Ansprüche 1 bis 10, gekennzeichnet durch eine Lichtquelle für ultraviolettes Licht, welche die Arbeitsplatte und die Verbrauchsmaterialien und Hilfsmittel mit keimtötendem Licht beleuchten kann.
